Europäisches Patentamt
European Patent Office
Office européen des brevets

(11) Publication number: **0 409 418 A1**

# (12) EUROPEAN PATENT APPLICATION

(21) Application number: 90306881.5

(22) Date of filing: 22.06.90

(51) Int. Cl.5 C07D 277/24, C07D 263/32, A01N 43/76, A01N 43/78

(30) Priority: 20.07.89 GB 8916574
14.02.90 GB 9003347

(43) Date of publication of application:
23.01.91 Bulletin 91/04

(84) Designated Contracting States:
AT BE CH DE DK ES FR GB GR IT LI LU NL SE

(71) Applicant: IMPERIAL CHEMICAL INDUSTRIES PLC
Imperial Chemical House, Millbank
London SW1P 3JF(GB)

(72) Inventor: Griffin, David Alan
10 Chivers Drive
Wokingham Berks RG11 4EW(GB)
Inventor: Elliott, Raymond
86 Egremont Drive, Lower Early
Reading Berks., RG6 3BS(GB)
Inventor: Lawson, Kevin Robert
High Stile, Piddington Lane
Piddington, High Wycombe, Bucks HP14 3BB(GB)
Inventor: Rice, Martin John
18 Le Marchant Road, Frimley
Camberley, Surrey GU16 5RW(GB)
Inventor: Hunt, Ian James
1 Rutland Court, Rutland Street
High Wycombe, HP11 2JS(GB)

(74) Representative: Ricks, Michael James et al
Imperial Chemical Industries PLC Legal Department: Patents PO Box 6 Bessemer Road
Welwyn Garden City Herts, AL7 1HD(GB)

## (54) Heterocyclic tertiary alcohols.

(57) Heterocyclic tertiary alcohol compounds useful as plant growth regulators have the general formula (I) :

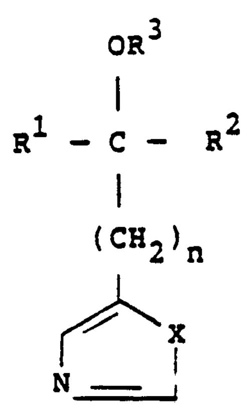

(I)

wherein n is an integer which is 0 or 1; X is S or O; $R^1$ is an optionally substituted secondary or tertiary alkyl group or an optionally substituted cycloalkyl or alkylcycloalkyl group containing a total of from 3 to 7 carbon atoms; $R^2$ is a group : $-(CH_2)_a-C\equiv C-A$ (II) or $-(CH_2)_b-CH=CH-A$ (III) or $-(CH_2)_c-A$ (IV) or $-CH_2-O-(CH_2)_d-A$ (V) wherein A is an optionally substituted aryl group, a, b and d are integers from 0 to 2 and c is an integer from 0 to 4; or $R^2$ is an optionally halo-substituted alkyl, cycloalkyl, cycloalkylalkyl alkylcycloalkyl group or alkylcycloal-

EP 0 409 418 A1

kylalkyl, or is an optionally halo-substituted alkenyl, cycloalkenyl, cycloalkenylalkyl, alkylcycloalkenyl or alkyl-cycloalkenylalkyl group, or is an optionally halo-substituted alkynyl group each of said groups containing a total of from 4 to 8 carbon atoms; $R^3$ is hydrogen, an alkyl group containing from 1 to 4 carbon atoms or an alkenyl or alkynyl group containing from 3 to 4 carbon atoms.

## HETEROCYCLIC TERTIARY ALCOHOLS

This invention relates to heterocyclic tertiary alcohol derivatives useful as plant growth regulating agents and fungicides, to processes for preparing them, to compositions containing them and to methods of regulating plant growth and combating fungal diseases in plants using them.

According to the present invention there is provided a heterocyclic tertiary alcohol compound having the general formula (I) :

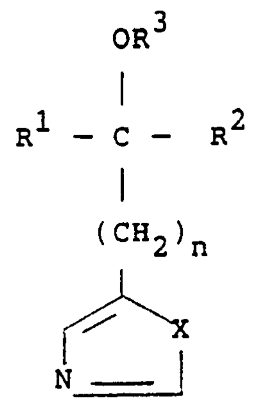

(I)

and stereoisomers thereof, wherein n is an integer which is 0 or 1; X is S or O; $R^1$ is an optionally substituted secondary or tertiary alkyl group containing from 3 to 7 carbon atoms or an optionally substituted cycloalkyl or alkylcycloalkyl group containing a total of from 3 to 7 carbon atoms; $R^2$ is a group :

$-(CH_2)_a-C\equiv C-A$ (II)

or

$-(CH_2)_b-CH=CH-A$ (III)

or

$-(CH_2)_c-A$ (IV)

or

$-CH_2-O-(CH_2)_d-A$ (V)

wherein A is an optionally substituted aryl group, a is an integer from 0 to 2, b is an integer from 0 to 2, c is an integer from 0 to 4 and d is an integer from 0 to 2; or $R^2$ is an optionally halo-substituted alkyl, cycloalkyl, cycloalkylalkyl alkylcycloalkyl group or alkylcycloalkylalkyl, each of said groups containing a total of from 4 to 8 carbon atoms; or is an optionally halo-substituted alkenyl, cycloalkenyl, cycloalkenylalkyl, alkylcycloalkenyl or alkylcycloalkenylalkyl group, each of said groups containing a total of from 4 to 8 carbon atoms; or is an optionally halo-substituted alkynyl group containing a total of from 4 to 8 carbon atoms; $R^3$ is hydrogen, an alkyl group containing from 1 to 4 carbon atoms, an alkenyl group containing from 3 to 4 carbon atoms or an alkynyl group containing from 3 to 4 carbon atoms; and agrochemically acceptable metal complexes of the compounds of formula (I) and esters (acylates) of compounds of formula (I) wherein $R^3$ is hydrogen.

The compounds of the invention contain one or more chiral centres. Such compounds are generally obtained in the form of racemic mixtures. However, these and other mixtures can be separated into the individual isomers by methods known in the art, and this invention embraces such isomers.

Optional substituents which may be present in the group $R^1$ are preferably halogen, for example chlorine or fluorine. Preferred groups $R^1$ are optionally substituted cyclopropyl or optionally substituted 1-methylcyclopropyl or the group:

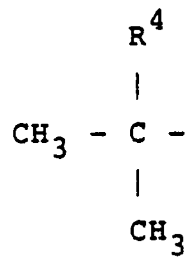

(VI)

wherein $R^4$ is hydrogen or an alkyl group containing from 1 to 4 carbon atoms, said group being optionally substituted either in the methyl groups or in $R^4$ when it is alkyl. $R^4$ is preferably hydrogen or an alkyl group containing 1 or 2 carbon atoms.

$R^1$ is preferably isopropyl, tertiary butyl or 1,1-dimethylpropyl.

When $R^2$ is an optionally halo-substituted cycloalkylalkyl or cycloalkenylalkyl group (that is a group in which the cycloalkyl or cycloalkenyl moiety is linked to the rest of the molecule through an alkyl chain), the optionally halo-substituted cycloalkyl or cycloalkenyl group preferably contains from 3 to 6, for example from 3 to 5 ring carbon atoms. The terms "alkylcycloalkylalkyl" and "alkylcycloalkenylalkyl" as used herein indicate a cycloalkylalkyl or cycloalkenylalkyl group respectively containing alkyl substitution in the cycloalkyl or cycloalkenyl ring.

Optional halogen substituents which may be present in the group $R^2$ include in particular chlorine, bromine or fluorine.

When $R^2$ is an optionally halo-substituted alkenyl group, it may have the cis or trans configuration and is preferably a group:

$-(CH_2)_m-CH=CH-R^5$ (VII)

where $R^5$ is hydrogen or an optionally halo-substituted alkyl group or is an optionally halo-substituted cycloalkyl or cycloalkylalkyl group, the total number of carbon atoms in the group $R^5$ and the value of the integer m being such that the total number of carbon atoms in the group $R^2$ is from 4 to 8. Especially preferred optionally halo-substituted alkenyl groups $R^2$ have the formula:

$-CH=CH-R^6$ (VIII)

wherein $R^6$ is hydrogen or an optionally halo-substituted alkyl group containing from 2 to 6 carbon atoms, especially from 3 to 4 carbon atoms.

When $R^2$ is an optionally halo-substituted alkynyl group it is preferably a group:

$-(CH_2)_p-C\equiv C-R^7$ (IX)

wherein $R^7$ is an optionally halo-substituted alkyl group or is an optionally halo-substituted cycloalkyl or cycloalkylalkyl group, the total number of carbon atoms in the group $R^7$ and the value of the integer p being such that the total number of carbon atoms in the group $R^2$ is from 4 to 8.

Especially preferred optionally halo-substituted alkynyl groups $R^2$ have the formula:

$-C\equiv C-R^8$ (X)

wherein $R^8$ is an optionally halo-substituted alkyl group containing from 2 to 6 carbon atoms, and especially from 3 to 4 carbon atoms.

Optional halogen substituents which may be present in the alkyl groups $R^5$, $R^6$, $R^7$ and $R^8$ include in particular chlorine, bromine and fluorine.

$R^3$ is preferably hydrogen or methyl.

The group A in formulae (II), (III), (IV) an (V) is preferably an optionally substituted phenyl group.

As examples of optional substituents which may be present in the phenyl group, A, there may be mentioned one or more substituents selected from halogen, for example chlorine, bromine or fluorine; alkyl, for example lower alkyl and especially methyl; alkoxy, for example lower alkoxy and especially methoxy; haloalkyl, for example lower haloalkyl and especially trifluoromethyl; nitro; and cyano. The term "lower" as applied to the above groups indicates that the group contains from 1 to 6, and preferably from 1 to 4 carbon atoms.

Thus as specific examples of the group A there may be mentioned phenyl, 2-, 3- and 4-chlorophenyl; 2-, 3- and 4-fluorophenyl; dichlorophenyl (for example 2,4-dichloro-phenyl); difluorophenyl (for example 2,4-difluorophenyl); 2-, 3- and 4-methylphenyl; 2-, 3- and 4-trifluoromethylphenyl; 2-, 3- and 4-methoxyphenyl; methoxychlorophenyl (for example 2-methoxy-4-chlorophenyl); and 2-, 3- and 4-nitrophenyl.

The preferred value of the integers, a, b and c in formulae (II), (III) and (IV) is 0 or 1; 0 is especially preferred.

The preferred value of the integer, d, in formula (III) is 0.

It is thus especially preferred that the group $R^2$ is:

-C≡C-A (II′)

or

-CH=CH-A (III′)

or

-A(IV′)

or

$-CH_2$-O-A (V′)

wherein A is as defined above.

The present invention includes metal complexes of the compounds of formula (I) and esters (acylates) of compounds of formula (I) wherein $R^3$ is hydrogen. As examples of esters there may be mentioned for example acetates or benzoates. Without limitation of the generality of the above statement, the present invention also includes any compound which breaks down in agrochemical use to a compound of formula (I).

Examples of the compounds of the invention are presented in Table I in which the values for $R^1$, $R^2$, $R^3$ X and n in the general formula (I) above are as indicated. Also to be considered as being specifically disclosed herein are the compounds wherein $R^2$, $R^3$, X and n take the values of Compound nos 2, 5, 6, 9, 10, 13, 14, 17, 18, 21, 22, 25, 26, 29 and 30 in Table 1 respectively and R' is t-pentyl (1,1-dimethyl-prop-1-yl) in place of t-butyl. Also to be considered as being specifically disclosed herein are compounds wherein $R^1$, $R^2$, X and n take the values of compound Nos 1 to 33 respectively in Table 1 and $R^3$ is methyl in place of hydrogen. Also to be considered as specifically disclosed herein are the compounds wherein $R^1$, $R^3$, X and n take the values of compound Nos 1 to 33 respectively, and $R^2$ is n-butyl in place of the respective values indicated in Table 1.

TABLE 1

| COMPOUND NUMBER | $R^1$ | $R^2$ | $R^3$ | X | n | MELTING POINT °C |
|---|---|---|---|---|---|---|
| 1 | t-butyl | $-C{\equiv}C-CH_2-CH(CH_3)_2$ | H | S | 0 | 81.2-82.8 |
| 2 | t-butyl | $-C{\equiv}C-CH_2-CH(CH_3)_2$ | H | O | 0 | 63-65 |
| 3 | isopropyl | $-C{\equiv}C-CH_2-CH(CH_3)_2$ | H | S | 0 | 69-70 |
| 4 | isopropyl | $-C{\equiv}C-CH_2-CH(CH_3)_2$ | H | O | 0 | |
| 5 | t-butyl | -C≡C-Phenyl | H | S | 0 | 110.4-112.8 |
| 6 | t-butyl | -C≡C-Phenyl | H | O | 0 | 132-134 |
| 7 | isopropyl | -C≡C-Phenyl | H | S | 0 | 98.0-100.6 |
| 8 | isopropyl | -C≡C-Phenyl | H | O | 0 | |
| 9 | t-butyl | $-C{\equiv}C-CH_2$Phenyl | H | S | 0 | |
| 10 | t-butyl | $-C{\equiv}C-CH_2-$Phenyl | H | O | 0 | |
| 11 | isopropyl | $-C{\equiv}C-CH_2-$Phenyl | H | S | 0 | |
| 12 | isopropyl | $-C{\equiv}C-CH_2-$Phenyl | H | O | 0 | |
| 13 | t-butyl | $-C{\equiv}C-(CH_2)_3-CH_3$ | H | S | 0 | 54.8-57.4 |
| 14 | t-butyl | $-C{\equiv}C-(CH_2)_3-CH_3$ | H | O | 0 | |
| 15 | isopropyl | $-C{\equiv}C-(CH_2)_3-CH_3$ | H | S | 0 | |
| 16 | isopropyl | $-C{\equiv}C-(CH_2)_3-CH_3$ | H | O | 0 | |
| 17 | t-butyl | $-C{\equiv}C-CH_2-CH(CH_3)_2$ | H | S | 1 | |

TABLE 1 (continued)

| COMPOUND NUMBER | $R^1$ | $R^2$ | $R^3$ | X | n | MELTING POINT °C |
|---|---|---|---|---|---|---|
| 18 | t-butyl | $-C\equiv C-CH_2-CH(CH_3)_2$ | H | O | 1 | |
| 19 | isopropyl | $-C\equiv C-CH_2-CH(CH_3)_2$ | H | S | 1 | |
| 20 | isopropyl | $-C\equiv C-CH_2-CH(CH_3)_2$ | H | O | 1 | |
| 21 | t-butyl | $-C\equiv C-$Phenyl | H | S | 1 | |
| 22 | t-butyl | $-C\equiv C-$Phenyl | H | O | 1 | 84.0-85.5 |
| 23 | isopropyl | $-C\equiv C-$Phenyl | H | S | 1 | |
| 24 | isopropyl | $-C\equiv C-$Phenyl | H | O | 1 | |
| 25 | t-butyl | $-C\equiv C-CH_2$Phenyl | H | S | 1 | |
| 26 | t-butyl | $-C\equiv C-CH_2-$Phenyl | H | O | 1 | |
| 27 | isopropyl | $-C\equiv C-CH_2-$Phenyl | H | S | 1 | |
| 28 | isopropyl | $-C\equiv C-CH_2-$Phenyl | H | O | 1 | |
| 29 | t-butyl | $-C\equiv C-(CH_2)_3-CH_3$ | H | S | 1 | |
| 30 | t-butyl | $-C\equiv C-(CH_2)_3-CH_3$ | H | O | 1 | |
| 31 | isopropyl | $-C\equiv C-(CH_2)_3-CH_3$ | H | S | 1 | |
| 32 | isopropyl | $-C\equiv C-(CH_2)_3-CH_3$ | H | O | 1 | |
| 33 | t-pentyl* | $-C\equiv C-CH_2-CH-(CH_3)_2$ | H | S | 0 | oil |

*t-pentyl indicates the group 1,1-dimethylprop-1-yl

TABLE 1 (continued)

| COMPOUND NUMBER | $R^1$ | $R^2$ | $R^3$ | X | n | MELTING POINT °C |
|---|---|---|---|---|---|---|
| 34 | t-butyl | $-(CH_2)_4CH_3$ | H | S | 0 | oil |
| 35 | t-butyl | $-(CH_2)_4CH_3$ | H | O | 0 | |
| 36 | t-butyl | $-CH_2-O-$ 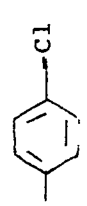 | H | S | 0 | gum |
| 37 | t-butyl | $-CH_2-O-$ 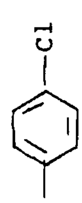 | H | O | 0 | |
| 38 | t-butyl | $-CH_2-O-$ 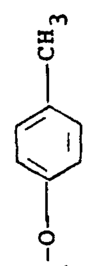 | H | S | 0 | gum |
| 39 | t-butyl | $-CH_2-O-$ 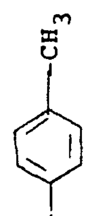 | H | O | 0 | |
| 40 | t-butyl | $-CH_2-O-$ 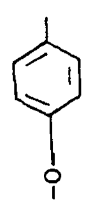 | H | S | 0 | gum |
| 41 | t-butyl | $-CH_2-O-$ 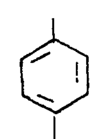 | H | O | 0 | |

Compounds of general formula (I) above wherein $R^3$ is hydrogen and X, n, $R^1$ and $R^2$ are as defined may be prepared by reacting a compound of general formula (XI) :

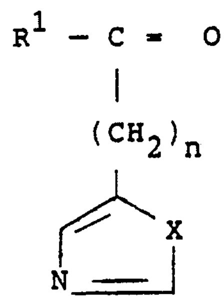

(XI)

where $R^1$, X and n have the values indicated for formula (I) with an organometallic compound which may be represented by the general formula (XII) :

$R^2M$ (XII)

where M is a suitable metal, for example lithium, magnesium or titanium and $R^2$ has the meaning given above.

The reaction conveniently takes place in a solvent such as diethylether, tetrahydrofuran or dichloromethane at -80° C to +80° C in an inert atmosphere. The product is obtained by quenching with a proton donor. When M is magnesium, the organometallic compound is more properly written as $R^2$-Mg-halogen. When M is titanium, the organometallic compound is more properly written as $R^2$-Ti(Oalkyl)$_3$.

The compounds of general formula (I) wherein $R^3$ is hydrogen, X is S and n is 0 may also be prepared by reacting a ketone of general formula (XIII), wherein $R^1$ and $R^2$ are as defined with an organometallic compound which may be represented by the general formula (XIV) wherein M is a suitable metal, for example lithium:

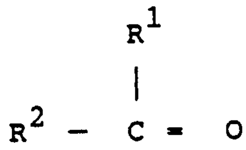

(XIII)

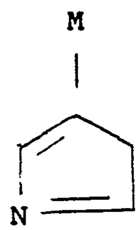

(XIV)

To ensure substitution of the metal M in the indicated 5-position, it may be necessary to block the 2-position with a suitable blocking group prior to formation of the organometallic compound. As an example of a suitable blocking group there may be mentioned the trimethylsilyl group, although other suitable blocking groups will occur to those skilled in the art. The blocking group in the 2-position may be removed if desired to form the compound of formula (XIV), but it is conveniently left in place during subsequent reaction and may be removed after the reaction is complete or may in fact be removed under the conditions of the subsequent reaction or purification.

The reaction preferably takes place in a suitable solvent such as diethyl ether or tetrahydrofuran at a temperature of from -120° C to +80° C and in an inert atmosphere. The product is obtained by quenching with a suitable proton donor.

The ketone intermediate of general formula (XI) above wherein n is 0 and X is S may be prepared by reacting an active ketone grouping (XV):

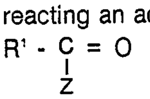 (XV)

wherein Z is an active group such as halogen, for example -Cl, alkoxy, for example methoxy or ethoxy, or $-N(OCH_3)(CH_3)$ with an organometallic group having the general formula (XIV) or a derivative thereof, which is protected in the 2- position as described above. The ketone intermediate of general formula (XI) above wherein n is 1 and X is S may similarly be prepared by reacting an active ketone grouping (XVI):

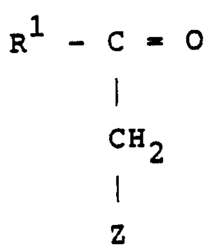

(XVI)

wherein Z has the meaning given above with an organometallic group of formula (XIV) or a protected derivative thereof. The ketone intermediate of general formula (XI) above wherein n is 1 and X is S may alternatively be prepared by reacting a compound of formula (XVII):

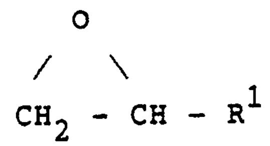

(XVII)

wherein $R^1$ has the meaning above with the organometallic derivative of formula (XIV) or a protective derivative thereof and oxidising the resultant alcohol to the ketone derivative of formula (XI). Any suitable oxidising agent may be used, and examples include chromate in a solvent such as pyridine.

The ketone intermediate of general formula (XI) above wherein n is 0 and X is O may be prepared in a cyclisation reaction in which a glyoxal of formula (XVIII) is reacted with a methlyisonitrile compound containing a leaving group, for example p-toluenesulphonylmethylisonitrile:

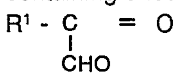 (XVIII)

The cyclisation suitably takes place in the presence of a base such as potassium carbonate and in a suitable solvent such as methanol or ethanol. The reaction suitably takes place at a temperature of between 0 °C and 100 °C.

The ketone intermediate of general formula (XI) above wherein n is 1 and X is O or S may also be prepared by the oxidation of an alcohol of formula (XIX) using conventional oxidation methods, for example the Swern oxidation:

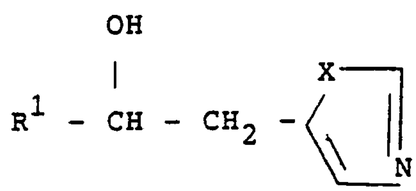

(XIX)

The alcohol of general formula (XIX) may be prepared in a cyclisation process by reaction of an amide of formula (XX):

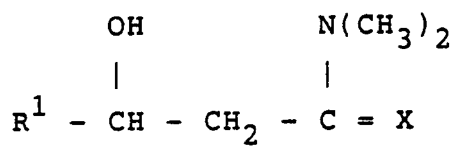

(XX)

with an organometallic derivative of methylisonitrile, for example the lithium derivative. The reaction conveniently takes place in a suitable solvent such as tetrahydrofuran, diethyl ether or dimethoxyethane at a temperature from -120 °C to 80 °C and in an inert atmosphere. The product is obtained by quenching with a suitable proton donor. In a modification of the above reaction, the compound of formula (I) wherein X is O or S and n is 1 can be obtained directly by cyclisation of a compound of formula (XXI):

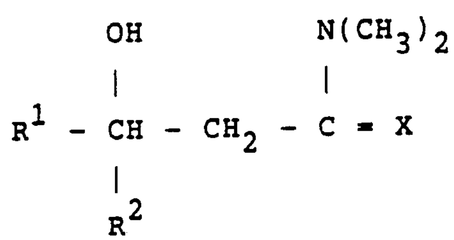

(XXI)

The reaction is analogous to the cyclisation of the compound of formula (XX).

Olefinic alcohols wherein $R^2$ is the group $-CH=CH-R^6$ wherein $R^6$ is as defined above may be made by the reduction of the corresponding acetylenic alcohol wherein $R^2$ is $-C \equiv C-R^6$, the group $R^6$ being the group

that it is desired to have in the olefinic product. If it is desired to produce a predominantly cis product, a suitable reducing agent is hydrogen in the presence of a suitable catalyst such as palladium on a support such as carbon (for example a Lindlar catalyst). If it is desired to product a predominantly trans product, a suitable reducing agent is a metal hydride reducing agent such as lithium aluminium hydride, "Red-Al" (sodium bis [2-methoxyethoxy] aluminium hydride) or sodium borohydride/palladium (II) chloride in a suitable solvent such as ether or tetrahydrofuran.

Similarly, compounds of formula (I) wherein R2 is a group (XXII):

$-CH_2CH_2-R^9$ (XXII)

where $R^9$ is an optionally substituted alkyl group such that the group (XXII) corresponds to the desired value of $R^2$, may be made by the complete reduction of the corresponding acetylenic alcohol, $-C\equiv C-R^9$. A suitable reducing agent is palladium, platinum or rhodium on a support such as carbon and in a suitable solvent such as methanol, ethanol or acetic acid.

Compounds of formula (I) wherein $R^2$ is the group (V) may be prepared by the reaction of an epoxide of formula (XXIII):

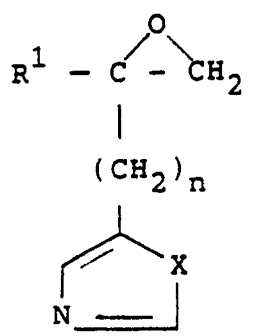

(XXIII)

with a suitable phenol in the presence of a base, for example sodium hydride, in a suitable solvent such as dimethylformamide.

Compounds of formula (XXIII) may be prepared by reaction of an organometallic compound of formula (XIV) or a derivative thereof, which is protected in the 2-position as described above with a compound for formula (XXIV):

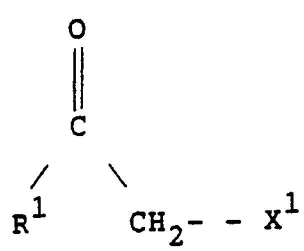

(XXIV)

wherein $X^1$ is halogen or another suitable leaving group.

The reaction preferably takes place in a suitable solvent such as diethylether (ether) or tetrahydrofuran in an inert atmosphere. The product is obtained by quenching with a suitable proton donor.

The ethers (wherein $R^3$ is alkyl) and esters (acylates) of the invention may be made from the corresponding hydroxy compound by reaction with the appropriate halide, acid chloride or acid anhydride in the presence of a suitable base.

The plant growth regulating effects of the compounds are manifested as, for example, by a stunting or dwarfing effect on the vegetative growth of woody and herbaceous mono- and di-cotyledcnous plants. Such stunting or dwarfing may be useful, for example, in peanuts, cereals such as wheat and barley, oil seed rape, field beans, sunflowers, potatoes and soya bean where reduction in stem height, with or without further advantageous effects such as stem strengthening, thickening and shortening, internode shortening, increased buttress root formation and more erect stem and leaf orientation, may reduce the risk of lodging and may also permit increased amounts of fertiliser to be applied. The stunting of woody species is useful in controlling the growth of undergrowth under power lines etc. Compounds which induce stunting or dwarfing may also be useful in modifying the stem growth of sugar cane thereby increasing the concentration of sugar in the cane at harvest; in sugar cane, the flowering and ripening may be controllable

by applying the compounds. Stunting of peanuts can assist in harvesting. Growth retardation of grasses can help maintenance of grass swards. Examples of suitable grasses are Stenotaphrum secundatum (St. Augustine grass), Cynosurus cristatus , Lolium multiflorum and perenne , Agrostis tenuis , Cynodon dactylon (Bermuda grass), Dactylis glomerata , Festuca spp. (eg, Festuca rubra ) and Poa spp. (eg, Poa pratense ). The compounds may stunt grasses without significant phytotoxic effects and without deleteriously affecting the appearance (particularly the colour) of the grass; this makes such compounds attractive for use on ornamental lawns and on grass verges. They may also have an effect on flower head emergence in, for example,grasses. The compounds can also stunt weed species present in the grasses; examples of such weed species are sedges (eg, Cyperus spp.) and dicotyledonous weeds (eg, daisy, plantain, knotweed, speedwell, thistle, docks and ragwort). The growth of non-crop vegetation (eg, weeds or cover vegetation) can be retarded thus assisting in the maintenance of plantation and field crops. In fruit orchards, particularly orchards subject to soil erosion, the presence of grass cover is important. However excessive grass growth requires substantial maintenance. The compounds of the invention could be useful in this situation as they could restrict growth without killing the plants which would lead to soil erosion; at the same time the degree of competition for nutrients and water by the grass would be reduced and this could result in an increased yield of fruit. In some cases, one grass species may be stunted more than another grass species; this selectivity could be useful, for example, for improving the quality of a sward by preferential suppression of the growth of undesirable species. The dwarfing may also be useful in miniaturising ornamental, household, garden and nursery plants (eg, poinsettias, chrysanthemums, carnations, tulips and daffodils).

As indicated above, the compounds can also be used to stunt woody species. This property can be used to control hedgerows or to shape or reduce the need for pruning, of fruit trees (eg, apples, pears, cherries, peaches, vines etc).

The plant growth regulating effect may (as implied above) manifest itself in an increase in crop yield; or in an ability in orchards and other crops to increase fruit set, pod set and grain set. Some coniferous trees are not significantly stunted by the compounds so the compounds could be useful in controlling undesirable vegetation in conifer nurseries. In the potato, vine control in the field and inhibition of sprouting in the store may be possible.

In addition the compounds may be useful as absicision agents resulting in thinning of fruit on the tree and an increase in fruit quality.

Other plant growth regulating effects caused by the compounds include alteration of leaf angle and changes in leaf morphology (both of which may permit increased light interception and utilisation) and promotion of tillering in monocotyledonous plants. Improved light interception is of value in all major world crops, eg, wheat, barley, rice, maize, soya, sugarbeet, potatoes, plantation crops and orchard crops. The leaf angle effect may be useful for example in altering the leaf orientation of, for example, potato crops thereby letting more light into the crops and inducing an increase in photosynthesis and tuber weight. By increasing tillering in monocotyledonous crops (eg, rice), the number of flowering shoots per unit area may be increased thereby increasing the overall grain yield of such crops. In addition better control and modification of hierarchical relationships is possible both in vegetative and reproductive stages of monocotyledonous and dicotyledons plant growth, especially in cereals such as wheat, barley, rice and maize, whereby the number of flowering shoots per unit area may be increased and the size distribution of grains within the ear may be modified in such a way as to increase yield. In the treatment of rice plants, or rice crops the invention compounds can be applied, eg, as granules or a granular formulation, for example as slow release granules, to nursery boxes, paddy water and other like cultivation loci and media. In grass swards, especially amenity grass, an increase in tillering could lead to a denser sward which may result in increased resilience in wear; and to increased yields and better quality of forage grass, eg, improved digestability and palatability.

The treatment of plants with the compounds can lead to the leaves developing a darker green colour. In dicotyledonous plants such as soyabean and cotton, there may be promotion of sideshooting.

The compounds may inhibit, or at least delay, the flowering of sugar beet (and thereby may increase sugar yield) or otherwise modify the flowering patterns in many other crops. They may also reduce the size of sugar beet without reducing significantly the sugar yield thereby enabling an increase in planting density to be made. Similarly in other root crops (eg, turnip, swede, mangold, parsnip, beetroot, yam and cassava) it may be possible to increase the planting density. The compounds could be useful in restricting the vegetative growth of cotton thereby leading to an increase in cotton yield. Crop yields may also be increased by improvement of the harvest index (ie. the harvested yield as a proportion of the total dry matter produced) by altering dry matter partitioning. This applies to all the aforementioned root, pod, cereal, tree, plantation and orchard crops.

The compounds may be useful in rendering plants resistant to stress since the compounds can delay the emergence of plants grown from seed, shorten stem height and delay flowering; these properties could be useful in preventing frost damage in countries where there is significant snow cover in the winter since then the treated plants would remain below snow cover during the cold weather. Further the compounds may cause drought or cold resistance in certain plants.

When applied as seed treatments at low rates the compounds can have a growth stimulating effect on plants.

It is to be understood that not all the compounds of the present invention will necessarily show all the above mentioned plant growth regulating effects. Thus whilst there may be advantages in compounds which have a broad spectrum of plant growth regulating effects against a wide range of species, compounds having a high specific activity with respect to a particular species and/or plant growth regulating effect may also be of great benefit.

In carrying out the plant growth regulating method of the invention, the amount of compound to be applied to regulate the growth of plants will depend upon a number of factors, for example the particular compound selected for use, and the identity of the plant species whose growth is to be regulated. However, in general an application rate of 0.1 to 15, preferably 0.1 to 5, kg per hectare is used. With the use of biodegradable polymeric slow release granules rates of 1 to 10g per hectare are feasible; whilst electrodynamic spraying techniques may also deploy lower rates of application. However, on certain plants even application rates within these ranges may give undesired phytotoxic effects. Routine tests may be necessary to determine the best rate of application of a specific compound for any specific purpose for which it is suitable.

The compounds may also be active fungicide and may be used to control one or more of the following pathogens :

Pyricularia oryzae on rice.

Puccinia recondita , Puccinia striiformis and other rust on wheat, Puccinia hordei , Puccinia striiformis and other rusts on barley, and rusts on other hosts, e.g. coffee, pears, apples, peanuts, vegetables and ornamental plants.

Erysiphe graminis (powdery mildew) on barley and wheat and other powdery mildews on various hosts such as Sphaerotheca macularis on hops, Sphaerotheca fuliginea on cucurbits (e.g. cucumber), Podosphaera leucotricha on Apple and Uncinula necator on vines.

Helminthosporium spp., Rhynchosporium spp., Septoria spp., Pseudocercosporella herepotrichoides and Gaeumannomyces graminis on cereals. Cercospora arachidicola and Cercosporidium personata on peanuts and other Cercospora species on other hosts, for example, sugar beet, bananas, soya beans and rice.

Botrytis cinerea (grey mould) on tomatoes, strawberries, vegetables, vines and other hosts.

Alternaria species on vegetables (e.g. cucumber), oil seed rape, apples, tomatoes and other hosts.

Venturia inaequalis (scab) on apples.

plasmopara viticola on vines.

Other downy mildews such as Bremia lactucae on lettuce, Peronospora spp. on soya beans, tobacco, onions and other hosts and Pseudoperonospora humuli on hops and Pseudoperonospora cubensis on cucurbits Phytophthora infestans on potatoes and tomatoes and other Phytophthora spp. on vegetables, strawberries, avocado, pepper, ornamentals, tobacco, cocoa and other hosts.

Thanaterphorus cucumeris on rice and other Rhizoctonia species on various host such as wheat and barley, vegetables, cotton and turf.

Some of the compounds show a broad range of activities against fungi in vitro . They may also have activity against various post-harvest diseases on fruit (e.g. Penicillium digitatum and Italicum and Trichoderma viride on oranges, Gloesporium musarum and bananas and Botrytis cinerea on grapes).

Further some of the compounds may be active as seed dressings against Fusarium spp., Septoria spp., Tilletia spp., (bunt, a seed borne disease of wheat), Ustilago spp., Helminthosporium spp. on cereals, Rhizoctonia solani on cotton and Pyricularia oryzae on rice.

The compounds may be used as such for plant growth regulating purposes or as fungicides but are more conveniently formulated into compositions for such usage. The invention thus provides a plant growth regulating or fungicidal composition comprising a compound of general formula (I) as hereinbefore defined, or a salt or metal complex thereof; and, optionally, a carrier or diluent.

The invention also provides a method of regulating plant growth or of fungicidal treatment, which comprises applying to the plant, to seed of a plant or to the locus of a plant or seed, a compound, or a salt or metal complex thereof, as hereinbefore defined, or a composition combining the same.

The compounds, metal complexes, ethers and esters can be applied in a number of ways, for example they can be applied, formulated or unformulated, directly to the foliage of a plant, or they can be applied

also to bushes and trees, to seeds or to other medium in which plants, bushes or trees are growing or are to be planted, or they can be sprayed on, dusted on or applied as a cream or paste formulation, or they can be applied as a vapour; or as slow release granules. Application can be to any part of the plant, bush or tree, for example to the foliage, stems, branches or roots, or to soil surrounding the roots, or to the seed before it is planted; or to the soil generally, to paddy water or to hydroponic culture systems. The invention compounds may also be injected into plants or trees and they may also be sprayed onto vegetation using electrodynamic spraying techniques.

The term "plant" as used herein includes seedlings, bushes and trees.

The compounds are preferably used for agricultural and horticultural purposes in the form of a composition. The type of composition used in any instance will depend upon the particular purpose envisaged.

The compositions may be in the form of dusting powders or granules comprising the active ingredient and a solid diluent or carrier, for example fillers such as kaolin, bentonite, kieselguhr, dolomite, calcium carbonate, talc, powdered magnesia, Fuller's earth gypsum, Hewitt's earth, diatomaceous earth and China clay. Such granules can be pre-formed granules suitable for application to the soil without further treatment. These granules can be made either by impregnating pellets of filler with the active ingredient or by pelleting a mixture of the active ingredient and powdered filler. Compositions for dressing seed, for example, may comprise an agent (for example a mineral oil) for assisting the adhesion of the composition to the seed; alternatively the active ingredient can be formulated for seed dressing purposes using an organic solvent (for example N-methylpyrrolidone or dimethylformamide).

The compositions may also be in the form of dispersible powders, granules or grains comprising a wetting agent to facilitate the dispersion in liquids of the powder or grains which may contain also fillers and suspending agents.

The aqueous dispersions or emulsions may be prepared by dissolving the active ingredient(s) in an organic solvent optionally containing wetting, dispersing or emulsifying agent(s) and then adding the mixture to water which may also contain wetting, dispersing or emulsifying agent(s). Suitable organic solvents are ethylene dichloride, isopropyl alcohol, propylene glycol, diacetone alcohol, toluene, kerosene, methylnaphthalene, the xylenes, trichloroethylene, furfuryl alcohol, tetrahydrofurfuryl alcohol, and glycol ethers (eg, 2-ethoxyethanol and 2-butoxyethanol).

The compositions to be used as sprays may also be in the form of aerosols wherein the formulation is held in a container under pressure in the presence of a propellant, eg, fluorotrichloromethane or dichlorodifluoromethane.

The compounds can be mixed in the dry state with a pyrotechnic mixture to form a composition suitable for generating in enclosed spaces a smoke containing the compounds.

Alternatively, the compounds may be used in a micro-encapsulated form. They may also be formulated in biodegradable polymeric formulations to obtain a slow, controlled release of the active substance.

By including suitable additives, for example additives for improving the distribution, adhesive power and resistance to rain on treated surfaces, the different compositions can be better adapted for various utilities.

The compounds can be used as mixtures with fertilisers (eg, nitrogen-, potassium- or phosphorus-containing fertilisers). Compositions comprising only granules of fertiliser incorporating, for example coated with, the compound are preferred. Such granules suitably contain up to 25% by weight of the compound. The invention therefore also provides a fertiliser composition comprising the compound of general formula (I) or a salt or metal complex thereof.

The compositions may also be in the form of liquid preparations for use as dips or sprays which are generally aqueous dispersions or emulsions containing the active ingredient in the presence of one or more surfactants eg, wetting agent(s), dispersing agent(s), emulsifying agent(s) or suspending agent(s); or which are spray formulations of the kind suitable for use in electrodynamic spraying techniques. The foregoing agents can be cationic, anionic or non-ionic agents. Suitable cationic agents are quaternary ammonium compounds, for example cetyltrimethylammonium bromide.

Suitable anionic agents are soaps, salts of aliphatic monoesters of sulphuric acid (for example sodium lauryl sulphate), and salts of sulphonated aromatic compounds (for example sodium dodecylbenzenesulphonate, sodium, calcium or ammonium lignosulphonate, butylnaphthalene sulphonate, and a mixture of sodium diisopropyl- and triisopropyl-naphthalene sulphonates).

Suitable non-ionic agents are the condensation products of ethylene oxide with fatty alcohols such as oleyl or cetyl alcohol, or with alkyl phenols such as octyl- or nonyl-phenol and octylcresol. Other non-ionic agents are the partial esters derived from long chain fatty acids and hexitol anhydrides, the condensation products of the said partial esters with ethylene oxide, and the lecithins. Suitable suspending agents are hydrophilic colloids (for example polyvinylpyrrolidone and sodium carboxymethylcellulose), and the vegetable gums (for example gum acacia and gum tragacanth).

The compositions for use as aqueous dispersions or emulsions are generally supplied in the form of a concentrate containing a high proportion of the active ingredient(s), and the concentrate is to be diluted with water before use. These concentrates often should be able to withstand storage for prolonged periods and after such storage be capable of dilution with water in order to form aqueous preparations which remain homogeneous for a sufficient time to enable them to be applied by conventional and electrodynamic spray equipment. The concentrates may conveniently contain up to 95%, suitably 10-85%, for example 25-60%, by weight of the active ingredient(s). These concentrates suitably contain organic acids (eg, alkaryl or aryl sulphonic acids such as xylenesulphonic acid or dodecyl benzenesulphonic acid) since the presence of such acids can increase the solubility of the active ingredient(s) in the polar solvents often used in the concentrates. The concentrates suitably contain also a high proportion of surfactants so that sufficiently stable emulsions in water can be obtained. After dilution to form aqueous preparations, such preparations may contain varying amounts of the active ingredient(s) depending upon the intended purpose, but an aqueous preparation containing 0.0005% to 10%, or 0.01% to 10%, by weight of active ingredient(s) may be used.

The compositions of this invention can comprise also one or more additional compound(s) having biological activity, eg, compounds having similar or complementary fungicidal or plant growth activity or compounds having plant growth regulating, herbicidal or insecticidal activity.

The additional fungicidal compound can be, for example, one which is capable of combating ear diseases of cereals (eg, wheat) such as Septoria , Gibberella and Helminthosporium spp., seed and soil borne diseases and downy and powdery mildews on grapes and powdery mildew and scab on apple etc. Examples of suitable additional fungicidal compound are imazalil, benomyl, carbendazim, thiophanate-methyl, captafol, captan, sulphur, triforine, dodemorph, tridemorph, pyrazophos, furalaxyl, ethirimol, tecnazene, dimethirimol, bupirimate, chlorothalonil, vinclozolin, procymidone, iprodione, metalaxyl, forsetyl-aluminium, carboxin, oxycarboxin, fenarimol, nuarimol, fenfuram, methfuroxan, nitrotal-isopropyl, triadimefon, thiabendazole, etridiazole, triadimenol, biloxazol, dithianon, binapacryl, quinomethionate, guazatine, dodine fentin acetate, fentin hydroxide, dinocap, folpet, dichlofluanid, ditalimphos, kitazin, cycloheximide, dichlobutrazol, a dithiocarbamate, a copper compound, a mercury compound, 1-(2-cyano-2-methoxyiminoacetyl)-3-ethyl urea, fenaponil, ofurace, propiconazole, etaconazole and fenpropemorph and fenpropidine.

The compounds of general formula (I) can be mixed with soil, peat or other rooting media for the protection of plants against seed-borne, soil-borne or foliar fungal diseases.

Suitable additional insecticides are Pirimor, Croneton, dimeth- oate, Metasystox, pyrethroid insecticides and formothion.

The other, additional, plant growth regulating compound can be one which controls weeds or seedhead formation, improves the level or longevity of the plant growth regulating activity of the compounds of general formula (I), selectively controls the growth of the less desirable plants (e.g. grasses) or causes the compound of general formula (I) to act faster or slower as a plant growth regulating agent. Some of these other agents will also be herbicides.

Examples of suitable plant growth regulating compounds, which can display synergy in admixture, or use, with the invention compounds are the gibberellins (eg, $GA_3$, $GA_4$ or $GA_7$), the auxins (eg, indoleacetic acid, indolebutyric acid, naphthoxyacetic acid or naphthylacetic acid), the cytokinins (eg, kinetin, diphenylurea, benzimidazole, benzyladenine or benzylaminopurine), phenoxyacetic acids (eg, 2,4-D or MCPA), substituted benzoic acids (eg, triiodobenzoic acid), morphactins (eg, chlorfluorecol), maleic hydrazide, glyphosate, glyphosine, long chain fatty alcohols and acids, dikegulac, fluoridamid, mefluidide, substituted quaternary ammonium and phosphonium compounds (eg, chlormequat* chlorphonium or mepiquat chloride*), ethephon, carbetamide, methyl-3,6-dichloroanisate, daminozide*, asulam, abscisic acid, isopyrimol, 1-(4-chlorophenyl)-4,6-dimethyl-2-oxo-1,2-dihydropyridine-3-carboxylic acid, hydroxybenzonitriles (eg, bromoxynil), difenzoquat*, benzoylprop-ethyl 3,6-dichloropicolinic acid, fenpentezol, triapenthanol, flurpirimidol, paclobutrazol, tetcyclacis and tecnazene. Synergy will be most likely to occur with those of the foregoing which are quaternary ammonium compounds and with those marked with an asterisk.

For certain applications, for example in the injection of the compounds of the invention into trees or plants, it is desirable that the compounds have a relatively high solubility in water, for example a solubility in excess of 30 parts per million. The compounds may alternatively be injected into the tree in the form of an organic solution, for example a solution in a lower alcohol.

For certain applications it is also desirable that the compound has a low persistency in soil to prevent carry-over to adjacent crops or even crops planted subsequently in the same soil. Preferably the compound for use in such applications has a half life in the soil of less than 20 weeks.

The invention is illustrated by the following Examples in which all parts and percentages are by weight unless otherwise stated.

EXAMPLE 1

This Example illustrates the preparation of 2,2,7-trimethyl-3-hydroxy-3-(thiazol-5-yl)oct-4-yne, Compound No 1 of Table 1.

Stage 1

Preparation of 2-(trimethylsilyl)thiazole.

To a solution of 2-bromothiazole (5.00g, 30 mmol) in dry diethylether (70 ml) at -78 °C under a nitrogen atmosphere was added n-butyllithium in hexane (12 ml of a 2.5 M solution, 30 mmol) maintaining the temperature below -70 °C. The mixture was stirred at -78 °C for a further 20 minutes. A solution of trimethylsilylchloride (4.24 ml, 34 mmol) in dry diethylether (10 ml) was added, ensuring that the temperature did not rise above -65 °C. The reaction mixture was stirred at -78 °C for a further two hours and then allowed to warm to room temperature. A saturated aqueous solution of ammonium chloride (10 ml) was added and the reaction contents allowed to stand overnight. The mixture was poured into a saturated aqueous solution of ammonium chloride and extracted with diethylether (3 x 100 ml). The combined organic layers were dried over magnesium sulphate and concentrated under reduced pressure to give a brown oily residue. The product was purified by distillation at atmospheric pressure (168-170 °C) to give a colourless oil.

Stage 2

Preparation of N-methoxy-N-methyl-2,2-dimethyl- propamide.

To a solution of 2,2-dimethylpropionylchloride (12.05 g, 0.1 mol) and N,O-dimethylhydroxylamine (9.755 g, 0.1 mol) in ethanol-free chloroform (120 ml) at 0 °C was added pyridine (17.38 g, 0.22 mol). The reaction mixture was allowed to warm to ambient temperature, stirred for one hour and then concentrated under reduced pressure. The solid residue was partitioned between brine and a 1:1 mixture of diethylether and dichloromethane. The combined organic layer was dried over magnesium sulphate and concentrated under reduced pressure to yield a colourless oil.

Stage 3

Preparation of 1-(thiazol-5-yl)-2,2-dimethylpropan-1-one.

To a solution of diisopropylamine (0.96 g, 9.5 mmol) in dry diethylether (20 ml) under a nitrogen atmosphere at -65 °C was added n-butyllithium in hexane (3.8 ml of a 2.5 M solution, 9.5 mmol). The mixture was stirred at -74 °C for one hour. A solution of 2-trimethylsilylthiazole prepared in Stage 1 (1.50 g, 9.5 mmol) in dry diethylether ( 5 ml) was added dropwise maintaining the temperature below -60 °C. After stirring the reaction mixture for a further two hours at -70 °C, a solution of N-methoxy-N-methyl-2,2-dimethylpropamide, prepared in Stage 2 (1.45 g, 10 mmol) in dry diethylether (5 ml) was added and the mixture allowed to warm to room temperature over a period of one hour. The reaction contents were poured into a saturated aqueous solution of ammonium chloride (100 ml) and extracted three times with diethylether. The combined organic layers were dried over magnesium sulphate and concentrated under reduced pressure to leave a yellow oily residue. The oil was subjected to column chromatography on silica, eluting with a 1:1 mixture of hexane and diethylether giving a two component mixture which was further

purified by vacuum distillation to give the desired product as an oil characterised by its NMR spectrum as follows:-
$^1$H NMR ($\delta$ $CDCl_3$, 270 MHz) 8.9 (1H, s); 8.5 (1H, s) 1.4 (9H, s)

Stage 4

To a solution of 4-methylpent-1-yne (0.815 g, 9.94 mmol) in dry tetrahydrofuran (40 ml) at 10 °C under a nitrogen atmosphere was added a solution of ethylmagnesium bromide in dry tetrahydrofuran (2.37 ml of a 3 M solution, 7.1 mmol). The mixture was stirred at this temperature for 30 minutes and then heated at reflux for a further 30 minutes. The reaction mixture was cooled to 0 °C and a solution of 1-(thiazol-5-yl)-2,2-dimethylpropan-1-one, prepared in Stage 4 (1.20 g, 7.1 mmol) in dry tetrahydrofuran (10 ml) was added. After warming to ambient temperature, the reaction contents were poured into a saturated aqueous solution of ammonium chloride and extracted three times with ethyl acetate. The combined organic layers were dried over magnesium sulphate and concentrated under reduced pressure to leave a brown oily residue. The oil was subjected to high pressure liquid chromatography (HPLC) purification on silica, eluting with a 99:1 mixture of chloroform and methanol giving the title compound as a crystalline solid (0.282 g) characterised by a melting point of 81.2-82.8 °C

EXAMPLE 2

This Example illustrates the preparation of 2,2-dimethyl-3-hydroxy-3-(thiazol-5-yl)-5-phenylpent-4-yne Compound No 5 of Table 1.

To a solution of phenylethyne (0.213 g, 2.09 mmol) in dry tetrahydrofuran (30 ml) at 10 °C under a nitrogen atmosphere was added a solution of ethylmagnesium bromide in dry tetrahydrofuran (0.58 ml of a 3M solution, 1.74 mmol). The mixture was stirred at this temperature for 30 minutes was cooled to 0 °C and a solution of 1-(thiazol-5-yl)-2,2-dimethylpropan-1-one (0.294 g, 1.74 mmol) (prepared as in Example 1) in dry tetrahydrofuran (10 ml) was added. After warming to ambient temperature, the reaction contents were poured into a saturated aqueous solution of ammonium chloride and extracted three times with ethyl acetate. The combined organic layers were dried over magnesium sulphate and concentrated under reduced pressure to leave a yellow oily residue. The oil was purified by HPLC on silica, eluting with a 99:1 mixture of chloroform and methanol giving the desired product as a colourless oil (0.282 g) which was characterised by its NMR spectrum as follows:-
$^1$H NMR ($\delta$ $CDCl_3$, 270 MHz) 8.7 (1H, s) 8.0 (1H, s) 7.3 (5H, m) 3.0 (1H, s) 1.2 (9H, s)

EXAMPLE 3

This Example illustrates an alternative method of preparation of 2,2-dimethyl-3-hydroxy-3-(thiazol-5-yl)-5-phenylpent-4-yne.

Stage 1

Preparation of 2,2-dimethyl-5-phenylpent-4-yne-3-one.

To a solution of pivaloyl chloride (10.0 g, 82 mmol) and phenylethyne (8.46 g, 82 mmol) in triethylamine (50 ml) at room temperature was added copper (I) iodide (0.15 g) and bis(triphenylphosphine)palladium (II) chloride (0.15 g). The reaction mixture was stirred at room temperature for 3 hours and then methanol (100 ml) was added. The solution was concentrated under reduced pressure and the residue was partitioned between diethylether and water. The aqueous layer was extracted three times with diethylether and the combined organic layers were dried over magnesium sulphate and concentrated under reduced pressure. The residue was purified by Kugelrohr distillation giving the product as an oil (boiling range 100-150 °C/1 mmHg).

Stage 2

To a solution of 2-trimethylsilylthiazole (1.27 g, 8.06 mmol) in diethylether (30 ml) at -78 °C was added a solution of n-butyllithium (3.22 ml of a 2.5 M solution in hexane, 8.06 mmol) and the solution was stirred for 1 hour. 2,2-Dimethyl-5-phenyl-4-pentyne-3-one prepared in Stage 1 (1.50 g, 8.06 mmol) in diethylether (5 ml) was added maintaining the temperature below -70 °C. The reaction mixture was allowed to warm to room temperature and then quenched with a saturated aqueous solution of ammonium chloride. The aqueous layer was washed twice with diethylether and the combined organic layers were dried over magnesium sulphate and concentrated under reduced pressure. The residue was purified by preparative HPLC on silica, eluting with 2% methanol in chloroform. The impure product was taken up in ethyl acetate and stirred in the presence of fresh silica. The solids were removed by filtration and the filtrate concentrated under reduced pressure to give the product as a white crystalline solid of melting point 110.4-112.8 °C. The product was also identified by the same NMR spectrum as the product of Example 2, notwithstanding the fact that the product of Example 2 failed to crystallise.

EXAMPLE 4

2-Methyl-3-hydroxy-3-(thiazol-5-yl)-5-phenylpent-4-yne (Compound No 7 of Table 1) was prepared using the general method of Example 3 by reaction of 2-methyl-5-phenylpent-4-yne-3-one with the organometallic compound derived from the reaction of 2-trimethylsilylthiazole with n-butyl lithium. The 2-methyl-5-phenylpent-4-yne-3-one was prepared by the reaction of isobutyryl chloride with phenylethyne in a process corresponding to that of stage 1 of Example 3.

The title product was a white crystalline solid of melting point 98.0-100.6 °C.

EXAMPLE 5

2,2-Dimethyl-3-hydroxy-3-(thiazol-5-yl)non-4-yne (Compound No 13 of Table 1) was prepared using the general method of Example 3 by reaction of 2,2-dimethylnon-4-yne-3-one with the organometallic compound prepared by reaction of 2-trimethylsilylthiazole with n-butyl lithium. 2,2-Dimethylnon-4-yne-3-one was prepared by the reaction of pivaloyl chloride with hex-1-yne in a process corresponding to that of stage 1 of Example 3.

The title product was a solid of melting point 54.8-57.4 °C.

EXAMPLES 6 AND 7

Compound Nos 3 and 33 were similarly prepared using the general method of Example 3. Compound No 3 was a solid melting at 68.8-70.2 °C, whilst Compound No 33 was an oil characterised by its NMR spectrum as follows:-

$^1H$ NMR (δ $CDCl_3$, 270 MHz) 8.7 (1H, s) 7.9 (1H, s) 2.7 (1H, s) 2.2 (2H, d) 1.9 (1H, multiplet) 1.5 (2H, quartet) 1.0 (12H, multiplet) 0.8 (3H, triplet)

EXAMPLE 8

This Example illustrates the preparation of 2,2-dimethyl-3-hydroxy-3-(oxazol-5-ylmethyl)-5-phenylpent-4-yne Compound No 22 of Table 1.

Stage 1

Preparation of 3-hydroxy-N,N,4,4-tetramethylpentanamide.

A solution of lithium diisopropylamide in cyclohexane (1.5M;37ml,50mmol) was added by syringe to tetrahydrofuran (50ml) stirred under nitrogen. The solution was cooled to -75 °C and N,N-dimethylacetamide (4.7ml,50mmol) was added. After 40 minutes, pivaldehyde (5.4ml,50mmol) was added and the reaction was quenched after a further 10 minutes by addition of excess acetic acid in tetrahydrofuran. Water was added and the resulting mixture was saturated with sodium chloride. The organic layer was separated and the aqueous phase extracted three times with ethyl acetate. The combined organic phases were dried over magnesium sulphate, filtered, and concentrated under reduced pressure to give a yellow oil (10g). The oil was distilled bulb to bulb at 1.5mmHg pressure and the fraction boiling at a bath temperature of 150 °C was collected. The product partly crystallised on standing and the crystals were washed with hexane to give a product of melting point 31-33 °C.

Stage 2

Preparation of 3,3-Dimethyl-1-(oxazol-5-yl)butan-2-ol.

To a stirred solution of methyl isocyanide (1.7ml,30mmol) in tetrahydrofuran (60ml) at -78 °C under nitrogen was added dropwise n-butyl lithium (2.5M in hexane;11.6ml,29mmol). After 15 minutes, 3-hydroxy-N,N,4,4-tetramethylpentanamide prepared in Stage 1 (865mg,5mmol) in tetrahydrofuran (10ml) was added, cooling the reaction flask with liquid nitrogen so as to keep the reaction temperature below -78 °C during the addition. After 10 minutes, the mixture was allowed to warm to -10 °C, quenched with methanol (6.5ml), and stirred for 1 hour. The solvent was removed under reduced pressure, the residue was triturated with ether, and the ether was removed under reduced pressure. The residue was partitioned between ethyl acetate and hydrochloric acid (2M;25ml) and the organic layer separated. The aqueous phase was further extracted twice with ethyl acetate and the combined organic phases were washed with saturated aqueous sodium hydrogen carbonate and with brine. The product was dried over magnesium sulphate, filtered, and concentrated under reduced pressure to give an oil. Column chromatography eluting with hexane/ethyl acetate (1:1) gave 3,3-dimethyl-1-oxazol-5-ylbutan-2-ol as an oil which crystallised after standing in the freezer overnight to give a solid of melting point 48-49 °C.

Stage 3

Oxidation of 3,3-Dimethyl-1-(oxazol-5-yl)butan-2-ol.

To a stirred solution of oxalyl chloride (236µl,2.79mmol) in dichloromethane (10ml) at -78 °C under nitrogen was slowly added dimethylsulphoxide (397µl,5.58mmol) followed, after 2 minutes, by 3,3-dimethyl-1-oxazol-5-ylbutan-2-ol prepared in Stage 2 (420mg,2.48mmol) in dichloromethane (5ml). After 15 minutes, triethylamine (1.71µl,12.4mmol) was added and, after a further 5 minutes, the reaction mixture was allowed to warm to room temperature. The mixture was partitioned between saturated aqueous sodium hydrogen carbonate and dichloromethane and the organic phase was separated. The aqueous layer was further extracted twice with dichloromethane and the combined organic layers were dried over magnesium sulphate, filtered, and concentrated under reduced pressure to give an oil. Column chromatography eluting with hexane/ethyl acetate (1:1) gave 3,3-dimethyl-1-oxazol-5-ylbutan-2-one as a mobile liquid.

Stage 4

Ethyl magnesium bromide (3M in tetrahydrofuran;3ml, 9mmol) was added to a stirred solution of phenyl acetylene (920µl,8.46mmol) in tetrahydrofuran (7ml) under nitrogen. The mixture was heated under reflux for 2 minutes and cooled to 5 °C. 3,3-Dimethyl-1-oxazol-5-ylbutan-2-one prepared in stage 3 (427mg,2.56mmol) in tetrahydrofuran (4ml) was added in one portion and the reaction was quenched after 5 minutes by addition of saturated aqueous ammonium chloride. The product was isolated by extraction (x3) with ethyl acetate, dried over magnesium sulphate, filtered, and concentrated under reduced pressure

giving a dark brown oil. Column chromatography eluting with hexane/ethyl acetate (6:4) gave the title compound as crystals of melting point 84-85.5 ° C.

EXAMPLE 9

This Example illustrates the preparation of 2,2,7-trimethyl-3-(oxazol-5-yl)oct-4-yn-3-ol (Compound No 2 of Table 1).

Stage 1

Preparation of 1-(oxazol-5-yl)-2,2-dimethylpropan-1-one.

To a stirred mixture of p-toluenesulphonyl-methylisonitrile (1.95g,10mmol) and potassium carbonate (1.4g,10mmol) in methanol (10ml) was added t-butylglyoxal (1.14g,10mmol). An exothermic reaction ensued which was completed by heating the mixture under reflux for 5 minutes. The reaction mixture was diluted with excess ethyl acetate and washed twice with water and brine. The product was dried over magnesium sulphate, filtered and concentrated under reduced pressure to give a yellow oil. Column chromatography eluting with hexane/ethyl acetate (2:1) gave 1-(oxaxol-5-yl)-2,2-dimethylpropan-1-one as an oil.

Stage 2

2,2,7-Trimethyl-3-(oxazol-5-yl)oct-4-yn-3-ol.

To a stirred solution of ethyl magnesium bromide (3M in tetrahydrofuran;2.5ml,7mmol) in tetrahydrofuran (5ml) under nitrogen was added isobutyl acetylene (0.6g,7mmol). The resulting mixture was heated under reflux for 10 minutes and cooled to 0 ° C. 1-(oxaxol-5-yl)-2,2-dimethyl-propan-1-one prepared in Stage 1 (0.5g,3.5mmol) in tetrahydrofuran (2ml) was added and the mixture was poured into saturated aqueous ammonium chloride. The aqueous solution was extracted three times with ethyl acetate and the combined organic phases were dried over magnesium sulphate, filtered and concentrated under reduced pressure to give a yellow oil. Column chromatography eluting with ethyl acetate/hexane (1:3) gave the title compound as a pale oil which crystallised on standing to give a solid of melting point 63-65 ° C.

EXAMPLE 10

This Example illustrates the preparation of 2,2-Dimethyl-3-oxazol-5-yl-5-phenylpent-4-yn-3-ol, compound No 6 of Table 1.

To a stirred solution of ethyl magnesium bromide (3M in tetrahydrofuran;3ml,9mmol) in tetrahydrofuran (5ml) under nitrogen was added phenyl acetylene (920µl,8.46mmol). The resulting mixture was heated under reflux for 5 minutes and cooled to 0 ° C. 1-(oxaxol-5-yl)-2,2-dimethylpropan-1-one, prepared as in Stage 1 of Example 7 (0.4g,2.8mmol) in tetrahydrofuran (2ml) was added and the mixture was poured into saturated aqueous ammonium chloride. The aqueous solution was extracted three times with ethyl acetate and the combined organic phases were dried over magnesium sulphate, filtered and concentrated under reduced pressure to give a dark oil. Column chromatography eluting with ethyl acetate/hexane (1:3) gave the title compound as orange crystals of melting point 132-134 ° C.

EXAMPLE 11

This Example illustrates the preparation of 2,2-dimethyl-3-(thiazol-5-yl)octan-3-ol (Compound No. 34 of

Table 1)

Step A : Preparation of 5-Bromothiazole

Method

2-Amino-5-bromothiazole (7.13g:39.8mmol) was dissolved in a mixture of o-Phosphoric acid (60ml) and nitric acid (11ml) and cooled to -5° C in an ice/acetone bath. Sodium nitrite (4.3g) in water (15ml) was added dropwise over 30 minutes keeping the temperature below 0° C. Stirring was continued for 15 minutes then hypophosphorous acid (22ml) was added dropwise over 30 minutes keeping the temperature below 0° C. Slow nitrogen evolution followed. The reaction was kept at this temperature for 3 hours and then allowed to warm to room temperature overnight. Significant amounts of a brown gas were evolved (probably bromine). The reaction mixture was neutralised with sodium hydroxide solution and steam distilled. The distillate was extracted with diethyl ether, the extracts dried over magnesium sulphate and concentrated under reduced pressure. The residue was distilled at 15mmHg and the fraction boiling between 61-65° C collected.

Yield 2.10g = 32%

$^1$H NMR : 8.8 (1H,s); 7.8 (1H,s)

Step B : Preparation of 2,2-dimethyl-3-(thiazol-5-yl)-octan-3-ol.

Method

The preparation was carried out under a nitrogen atmosphere. 5-Bromothiazole (0.5g;3.04mmol) in dry diethyl ether (2ml) was added to a solution of n-butyl lithium (1.22ml of 2.5M in hexanes;3.05mmol) in diethyl ether (10ml) at -78° C. A red precipitate was formed and the suspension was stirred for a further 2 minutes. 2,2-dimethyl-octan- 3-one (0.5g;3.79mmol) in diethyl ether (2ml) was added to the reaction mixture over 2 minutes and the solution was allowed to warm to room temperature over 6 hours. Water was added and the solution extracted twice with ether. The combined extracts were washed with water, dried over magnesium sulphate and concentrated under reduced pressure. The oily residue was purified by HPLC on silica gel (ether/hexane 1:1) to give the product (Compound No. 1) as an oil.

Yield 0.45g = 61%

$^1$H NMR : 8.7 (1H,s); 7.6 (1H,s); 1.9 (2H,m); 1.3 (6H,m); 1.0 (9H,s); 0.9 (3H,m)

MS : 241($M^+$), 223, 208, 184 (100%), 127

## EXAMPLE 12

Preparation of 2,2-dimethyl-3-(thiazol-5-yl)non-4-yn-3-ol (Compound No. 13 of Table 1)

Step A : Preparation of 2,2-dimethylnon-4-yn-3-one

Method

The preparation was carried out under a nitrogen atmosphere. Pivaloyl chloride (10.0g;0.082mol) and 1-hexyne (6.72g; 0.082mol) were stirred together in triethylamine (40ml) at room temperature. Copper iodide (0.15g) and bis(triphenylphospine) palladium (II) chloride (0.15g) were added and the reaction mixture stirred for approximately 4 hours before methanol (30ml) was added. The reaction mixture was concentrated under reduced pressure to yield a black/brown solid, which was partitioned between diethyl ether and water. The organic layer was collected and the aqueous layer washed twice with diethyl ether. The combined

organic layers were dried over magnesium sulphate before concentrating under reduced pressure to give a brown oil. The product was purified by Kugelrohr distillation (60-100 °C, 0.2mmHg).
Yield 3.9g = 29%
$^1$H NMR : 2.4 (2H,t); 1.5 (4H,m); 1.2 (9H,s); 0.9 (3H,t.)
MS : 167 ($MH^+$), 166 ($M^+$), 151, 137, 123, 109 (100%)

Step B : Preparation of 2-Trimethylsilylthiazole

Method

The preparation was performed under a nitrogen atmosphere. n-Butyl lithium (24.4ml of 2.5M in hexanes;0.061mol) was added dropwise to a stirred solution of 2-bromothiazole (10g;0.061mol) in diethyl ether (140ml) at -72 °C. The solution was stirred for 40 minutes at this temperature after which time the trimethylsilyl chloride (8.74ml) in diethyl ether (20ml) was added (the temperature rose to approximately -65 °C). The reaction mixture was stirred at -72 °C for two hours before allowing it to warm to room temperature overnight. The reaction mixture was poured onto saturated ammonium chloride solution, before being extracted 3 times with diethyl ether. The combined organic layers were dried over magnesium sulphate before being concentrated under reduced pressure. The product was purified by distillation at atmospheric pressure collecting the fraction boiling between 165-170 °C.
Yield 5.09g = 53%
$^1$H NMR : 8.1 (1H,d); 7.5 (1H,d); 0.4 (9H,s)
MS : 157($M^+$), 142 (100%), 115

Step C : Preparation of 2,2-dimethyl-3-(thiazol-5-yl)-non-4-yn-3-ol

Method

The preparation was carried out under a nitrogen atmosphere. n-Butyl lithium (3.62ml of 2.5M solution;9.04mmol) was added to a solution of 2-trimethylsilylthiazole (1.42g; 9.04mmol) in diethyl ether (30ml) at -78 °C. After 1 hour at this temperature the 2,2-dimethylnon-4-yn-3-one (1.5g;9.04mmol) in diethyl ether (5ml) was added (at -78 °C) and the reaction mixture allowed to warm to room temperature. Saturated ammonium chloride solution was added and the solution extracted 3 times with diethyl ether.

The combined organic layers were dried over magnesium sulphate before concentrating under reduced pressure to yield a yellow oil. This was purified by HPLC on silica gel (eluting with diethyl ether/hexane 3:1).
Yield 0.876g = 39%
$^1$H NMR : 8.7 (1H,s); 7.8 (1H,s); 3.0 (1H,s); 2.3 (2H,t); 1.5 (4H,m); 1.0 (9H,s); 0.9 (3H,t)
MS : 251 (M+), 194 (100%), 152, 112

EXAMPLE 13

Preparation of 2,2-dimethyl-4-(4-chlorophenoxy)-3-(thiazol-5-yl)butan-3-ol (Compound No. 36 of Table 1)

Step A : Preparation of 2-t-butyl-2-(thiazol-5-yl)oxirane

Method

The preparation was carried out under a nitrogen atmosphere. n-Butyl lithium (11.2ml of 2.5M solution in hexanes;28mmol) was added to a solution of 2-trimethylsilylthiazole (4.42g: 28mmol) (Example 2 Step B)

in diethyl ether (100 ml) at -78° C. After stirring at -78° C for 1 hour, 1-bromo-3,3-dimethylbutan-2-one (5.01g; 28mmol) in diethyl ether (10ml) was added. The temperature was kept below -70° C. Once the addition was complete the reaction mixture was allowed to warm to room temperature, before adding saturated ammonium chloride solution. The solution was extracted with diethyl ether 3 times. The combined organic layers were dried over magnesium sulphate before being concentrated under reduced pressure to yield a yellow/brown oil. This was purified by HPLC on silica gel (eluting with hexane/ diethyl ether 1:1).
Yield 2.86g = 56%
$^1$H NMR : 8.7 (1H,s); 7.8 (1H,s); 3.2 (1H,d); 2.7 (1H,d); 1.0 (9H,s)
MS : 184 ($MH^+$), 156, 127, (100%), 98, 57

Step B : Preparation of 2,2-dimethyl-4-(4-chlorophenoxy)3-(thiazol-5-yl)butan-3-ol

Method

The preparation was carried out under a nitrogen atmosphere. Sodium hydride (0.178g of 55% dispersion in oil;4.095mmol) was washed with hexane before stirring with 10ml of dimethylformamide. p - Chlorophenol (1.4g; 10.92mmol) in dimethylformamide (10ml) was added to the reaction mixture (effervescence was observed). 2-t-butyl-2-(thiazol-5-yl) oxirane (0.5g; 2.73mmol) in dimethylformamide (5ml) was added to the reaction mixture which was then heated at 80° C for 5 hours. The mixture was cooled and left at room temperature overnight.

The reaction mixture was partitioned between 2N hydrochloric acid and diethyl ether with the aqueous layer being washed with diethyl ether. The combined organic layers were washed with 10% sodium hydroxide, before drying over magnesium sulphate and then concentrated under reduced pressure to yield a yellow oil.

Some product was obtained by triturating with hexane (solid precipitate). However, mother liquors of trituration were combined with the rest of the oil and purified by HPLC on silica gel (eluted with diethyl ether/hexane 3:1).
Yield 0.143 = 17.1%
$^1$H NMR : 9.0 (1H,s); 7.8 (1H,s); 7.2 (2H,d); 6.9 (2H,d), ($d_6$ DMSO) 4.4 (1H,d); 4.1 (1H,d); 0.9 (9H,s)
MS : 311 ($M^+$), 354, 170, 128 (100%)

EXAMPLE 14

2,2-Dimethyl-4-(4-methylphenoxy)-3-(thiazol-5-yl) butan-3-ol (Compound No. 38 of Table 1) was prepared using the same general method as Example 13 and was a yellow/brown oil. $^1$H NMR : 8.7 (1H,s); 7.7 (1H,s); 7.1 (2H,d); 6.7 (2H,d); 4.3 (2H,s); 3.4 (1H,s); 2.3 (3H,s); 1.1 (9H,s)
MS : 291 ($M^+$), 234, 184, 170, 128 (100%)

EXAMPLE 15

2,2-Dimethyl-4-phenoxy-3-(thiazol-5-yl)-butan-3-ol (Compound No. 40 of Table 1) was prepared using the same general method as Example 13 and was a yellow/brown oil.
$^1$H NMR : 8.7 (1H,s); 7.7 (1H,s); 7.3 (2H,m); 7.0 (1H,t.); 6.8 (2H,d); 4.3 (2H,s); 3.4 (1H,s); 1.1 (9H,s)
MS : 277 ($M^+$), 220, 184, 170, 128 (100%)

The manner in which the compounds of the present invention may be formulated into compositions suitable for application is shown generally in the following indicative illustrations numbered as Examples 16 to 25.

EXAMPLE 16

An emulsifiable concentrate is made up by mixing the following ingredients, and stirring the mixture

until all the constituents are dissolved.

| | |
|---|---|
| Compound of Table I | 10% |
| Calcium dcdecylbenzenesulphonate | 5% |
| "SYNPERONIC" NP13 | 5% |
| "Aromasol" H | 80% |

EXAMPLE 17

A composition in the form of grains readily dispersible in a liquid, e.g.. water, is prepared by grinding together the first three ingredients in the presence of added water and then mixing in the sodium acetate. The resultant mixture is dried and passed through a British Standard mesh sieve, size 44-100, to obtain the desired size of grains.

| | |
|---|---|
| Compound of Table I | 50% |
| "Dispersol" T. | 25% |
| "SYNPERONIC" NP5 | 1.5% |
| Sodium acetate | 23.5% |

EXAMPLE 18

The following ingredients are ground together to produce a powder formulation readily dispersible in liquids.

| | |
|---|---|
| Compound of Table I | 45% |
| "Dispersol" T. | 5% |
| "SYNPERONIC" NX | 0.5% |
| "Cellofas" B600 | 2% |
| China clay GTY powder | 47.5% |

EXAMPLE 19

The active ingredient is dissolved in acetone and the resultant liquid is sprayed on to the granules of attapulgite clay. The solvent is then allowed to evaporate to produce a granular composition.

| | |
|---|---|
| Compound of Table I | 5% |
| Attapulgite granules | 95% |

EXAMPLE 20

A composition suitable for use as a seed dressing is prepared by mixing the three ingredients.

| | |
|---|---|
| Compound of Table I | 50% |
| Mineral oil | 2% |
| China clay | 48% |

EXAMPLE 21

A dusting powder is prepared by mixing the active ingredient with talc.

| | |
|---|---|
| Compound of Table I | 5% |
| Talc | 95% |

EXAMPLE 22

A flowable formulation is prepared by bead-milling the constituents set out below and then forming an aqueous suspension of the ground mixture with water.

| | |
|---|---|
| Compound of Table I | 40% |
| "Dispersol" T. | 4% |
| "SYNPERONIC" NP5 | 1% |
| Water | 55% |

EXAMPLE 23

A dispersible powder formulation is made by mixing together the ingredients set out below and then grinding the mixture until all are thoroughly mixed.

| | |
|---|---|
| Compound of Table I | 25% |
| "Aerosol" AT/B | 2% |
| "Dispersol" AK. | 5% |
| China clay | 28% |
| Silica | 40% |

EXAMPLE 24

This Example illustrates the preparation of a dispersible powder formulation. The ingredients are mixed and the mixture then ground in a comminution mill.

| | |
|---|---|
| Compound of Table I | 25% |
| "PERMINAL" BX | 1% |
| "Dispersol" T. | 5% |
| Polyvinylpyrrolidone | 10% |
| Silica | 25% |
| China clay | 34% |

EXAMPLE 25

The ingredients set out below are formulated into dispersible powder by mixing then grinding the ingredients.

| | |
|---|---|
| Compound of Table I | 25% |
| "Aerosol" AT/B | 2% |
| "Dispersol" A | 5% |
| China clay | 68% |

There now follows an explanation of the compositions or substances represented by the various Trade Marks and Trade Names mentioned above.
"SYNPERONIC" NP13 : a condensate of nonyl phenol (1 mole) with ethylene oxide (13 moles).
"AROMASOL" H : a solvent mixture of alkylbenzenes.
"DISPERSOL" T. AND OAK : a mixture of sodium sulphate and a condensate of formaldehyde with sodium naphthalene sulphonate.
"SYNPERONIC" $NP_5$ a condensate of nonyl phenol (1 mole) with naphthalene oxide (5.5 moles).
CELLOFAS B600 : a sodium carboxymethyl cellulose thickener

EXAMPLE 26

Certain compounds in Table I (as indicated below) were tested for plant growth regulator activity against two species for various growth effects relevant to plant growth regulation.

Methodology

The plant species used in this screen are presented in Table II with the leaf stage at which they were sprayed. Except where indicated in Table III, each chemical was applied at 4000 ppm (4 kg/he in a 1000 l/ha field volume) or 3000 ppm respectively using a tracksprayer and a 6502 (Teejet) nozzle. After spray the plants were grown in a glasshouse with 25 °C day/22 °C night temperatures and supplementary lighting was supplied when necessary (from mercury vapour lamps), to provide a 16 hour photoperiod. The exception to this were the temperate cereals, wheat and barley which grown in 16 °C day/13 °C night temperatures.

After 2-6 weeks in the glasshouse, depending on the time of year, the plants were visually assessed for morphological characteristics. Formulation blanks were used as controls to assess the plants. The results are presented in Table III.

TABLE II

| PLANT MATERIAL USED FOR WHOLE PLANT SCREEN | | | | | |
|---|---|---|---|---|---|
| Species | Code | Variety | Growth Stage at Treatment | No. Plants per 3" Pot | Compost Type |
| Wheat | WW | Rapier | 1-1.5 leaves | 4 | JIP* |
| Tomato | TO | Ailsa Craig | 2-2.5 leaves | 1 | JIP |

JIP* = John Innes Potting Compost.

TABLE III

| COMPOUND NO. | RATE (PPM) | TO | | | | | WW | | | | |
|---|---|---|---|---|---|---|---|---|---|---|---|
| | | R | G | A | T | I | R | G | A | T | I |
| 1 | 4000 | | | | | | 3 | 1 | | 2 | 3 |
| 3 | 4000 | | | | | | 2 | | | | 2 |
| 5 | 3000 | 1 | | | | 1 | 3 | 1 | | 1 | 3 |
| 7 | 4000 | | | | | | 1 | | | 1 | 1 |
| 13 | 4000 | | | 2 | 1 | | 2 | | | | 2 |
| 33 | 4000 | | | 1 | 1 | | 2 | | | | 2 |
| 34 | 2000 | | | | | | 1 | | | | 1 |
| 36 | 4000 | | | 1 | | | 2 | | | | 2 |
| 38 | 4000 | | | 1 | | | 1 | | | | 1 |

Key :

R = Retardation
G = Greening effect
A = Apical damage
T = Tillering or side shooting
I = Interligular or internodal length reduction

All effects are scored visually on a 1-3 basis where
1 = 10-30%
2 = 31-60%
3 = 61-100%

EXAMPLE 27

Intermediate Retardant Test

Methodology

Three species are involved in this test RICE, SPRING-BARLEY and OILSEED RAPE (Brassica napus) . The variety and growth stages at spray are outlined in Table IV. Compounds were applied at 2000 ppm (2 kg $ha^{-1}$ at a field volume of 1000 l $ha^{-1}$). For rice and barley, the application was as an overall spray which

gives a foliar and root component in the test, ie, this test will detect the activity of both root and foliar acting compounds. For oilseed rape the effect of root acting and foliar acting compounds was examined separately in an attempt to detect relatively rare foliar activity. Thus for this species, separate root drench applications and foliar only sprays were each applied at a rate of 2 kg $ha^{-1}$.

The rice was grown in 4" 'paddy' pots, ie, the roots and bottom of the stems are immersed in water under conditions corresponding to those in paddy fields. Spring barley was grown in 4" pots. The plants were assessed for height to top-most ligule at approximately 21 days after treatment for spring barley and rice and for height to apex at approximately 21 days after the treatment for rape. In each case the result for each compound is compared to the height of the formulation blank in that test and presented as a percentage reduction in height compared to the formulation blank. A blank indicates that the compound was substantially inactive as a retardant at that particular rate of application. The results are presented in Tables V-VII.

TABLE IV

| Plant Material for Intermediate Retardant Test | | | | |
|---|---|---|---|---|
| SPECIES | VARIETY | GROWTH STAGE AT TREATMENT | NO. PLANTS PER 4" POT | Compost |
| Spring Barley | Atem | 3 leaves | 3 | JIP1 |
| Rice | Ishikari | 3 - 4 leaves | 2 | GRIT : JIP 3 |
| Oilseed Rape (Brassica napus) | | 3 - 4 leaves | 1 | JIP 1 |
| JIP 1 = John Innes Potting Compost. | | | | |

TABLE V

| RICE | |
|---|---|
| % Reduction (Compared to Formulation Blank) | |
| COMPOUND NO. | 2000 g/ha |
| 1 | 51 |

TABLE VI

| BARLEY | |
|---|---|
| % Reduction (Compared to Formulation Blank) | |
| COMPOUND NO. | 2000 g/ha |
| 1 | 56 |

TABLE VII

| OILSEED RAPE | | |
|---|---|---|
| % Reduction (Compared to Formulation Blank) | | |
| COMPOUND NO. | Foliar Only 2000 g/ha | Root Drench 2000 g/ha |
| 1 | 3 | 20 |

## Claims

1. A heterocyclic tertiary alcohol compound having the general formula (I) :

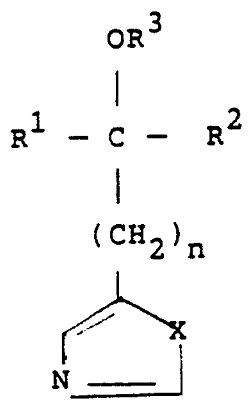

(I)

and stereoisomers thereof, wherein n is an integer which is 0 or 1; X is S or O; $R^1$ is an optionally substituted secondary or tertiary alkyl group containing from 3 to 7 carbon atoms or an optionally substituted cycloalkyl or alkylcycloalkyl group containing a total of from 3 to 7 carbon atoms; $R^2$ is a group :
$-(CH_2)_a-C\equiv C-A$ (II)
or
$-(CH_2)_b-CH=CH-A$ (III)
or
$-(CH_2)_c-A$ (IV)
or
$-CH_2-O-(CH_2)_d-A$ (V)
wherein A is an optionally substituted aryl group, a is an integer from 0 to 2, b is an integer from 0 to 2, c is an integer from 0 to 4 and d is an integer from 0 to 2;
or $R^2$ is an optionally halo-substituted alkyl, cycloalkyl, cycloalkylalkyl alkylcycloalkyl group or alkylcycloalkylalkyl, each of said groups containing a total of from 4 to 8 carbon atoms; or is an optionally halo-substituted alkenyl, cycloalkenyl, cycloalkenylalkyl, alkylcycloalkenyl or alkylcycloalkenylalkyl group, each of said groups containing a total of from 4 to 8 carbon atoms; or is an optionally halo-substituted alkynyl group containing a total of from 4 to 8 carbon atoms; $R^3$ is hydrogen, an alkyl group containing from 1 to 4 carbon atoms, an alkenyl group containing from 3 to 4 carbon atoms or an alkynyl group containing from 3 to 4 carbon atoms; and agrochemically acceptable metal complexes of the compounds of formula (I) and esters (acylates) of compounds of formula (I) wherein $R^3$ is hydrogen.

2. A compound according to claim 1 wherein $R^1$ is an optionally halo-substituted group:

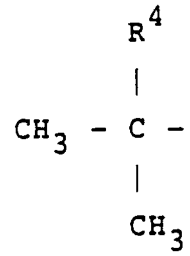

(VI)

wherein $R^4$ is hydrogen or an alkyl group containing from 1 to 4 carbon atoms.

3. A compound according to claim 1 or 2 wherein $R^2$ is a group of formula (II′) or (V′)

-C≡C-A (II′)

$-CH_2-O-A$ (V′)

wherein A is phenyl optionally substituted by one or more substituents selected from halogen, lower alkyl, lower alkoxy, lower haloalkyl, nitro and cyano; or

$R^2$ is an optionally halo-substituted alkynyl group of formula

$-C \equiv C-R^8$ IX)

wherein $R^8$ is an optionally halo-substituted alkyl group containing form 3 to 4 carbon atoms.

4. A compound according to any of the preceding claims wherein $R^3$ is hydrogen.

5. A method of preparing a compound of formula (I) in claim 1 wherein $R^3$ is hydrogen and X, n, $R^1$ and $R^2$ are as defined in claim 1 which comprises:

i) reacting a compound of general formula (XI) :

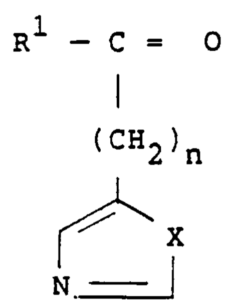

(XI)

with an organometallic compound which may be represented by the general formula (XII) : $R^2M$ (XII) where M is a suitable metal; or

ii) when X is S and n is 0, reacting a ketone of general formula (XIII), with an organometallic compound which may be represented by the general formula (XIV) wherein M is a suitable metal, for example lithium:

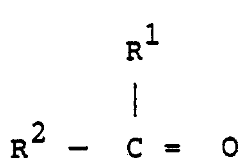

(XIII)

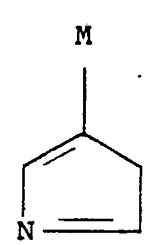

(XIV)

and wherein the 2- position in the organometallic compound of formula (XIV) is optionally blocked with a suitable blocking group prior to formation of the organometallic compound; or

iii) wherein n is 1, cyclising a compound of formula (XXI):

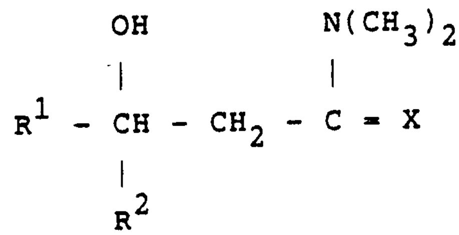 (XXI)

or

iv) wherein $R^2$ is the group (V) as defined in claim 1, reacting an epoxide of formula (XXIII):

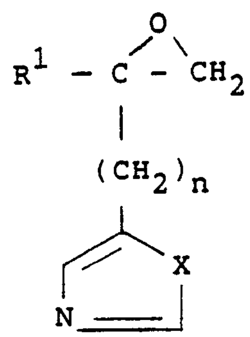 (XXIII)

with a suitable phenol in the presence of a base and a suitable solvent.

6. A plant growth regulating composition comprising a plant growth regulating amount of a compound according to claims 1 and an agrochemically acceptable carrier or diluent.

7. A method of regulating plant growth which comprises applying to the plant, to the seed of the plant or to the locus of the plant, a plant growth regulating amount of a compound according to claim 1.

European Patent Office

# EUROPEAN SEARCH REPORT

Application Number

EP 90306881.5

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | FR - A1 - 2 555 583 (INSTITUT DE RECHERCHES QUIMIQUES ET BIOLOGIQUES ATLIQUEES) * Claims 1,7 * -- | 1,5 | C 07 D 277/24<br>C 07 D 263/32<br>A 01 N 43/76<br>A 01 N 43/78 |
| A | EP - A2 - 0 096 890 (TAKEDA CHEMICAL INDUSTRIES) * Abstract * ---- | 1,5 | |
| | | | TECHNICAL FIELDS SEARCHED (Int. Cl.5) |
| | | | C 07 D 277/00<br>C 07 D 263/00<br>A 01 N 43/00 |

The present search report has been drawn up for all claims

| Place of search | Date of completion of the search | Examiner |
|---|---|---|
| VIENNA | 11-10-1990 | BRUS |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document